# EUROPÄISCHE PATENTANMELDUNG

(11) **EP 4 844 351 A1**
(43) Veröffentlichungstag der Anmeldung: **25.11.2026**
(21) Anmeldenummer: 26173096.4
(22) Anmeldetag: 17.04.2026
(51) Int. Cl.: G01J 1/42, A61B 90/30, G01J 3/50, G01J 1/02, G01J 3/28

(54) **KAMERABASIERTE SCHATTERMESSUNG MIT DIGITAL PROJIZIERTEN ABSCHATTERN**

(30) Priorität: 05.05.2025 DE 102025117033
(71) Anmelder: optical design unit gmbh, 6068 Mils (AT)
(72) Erfinder: Tanler, Peter, 6071 Aldrans (AT)
(74) Vertreter: Frenkel, Matthias Alexander

(57) **Zusammenfassung**

Vorgestellt wird eine technische Lösung, in der von einer Kamera generierte Bilddaten zur Berechnung einer Referenz-Helligkeit und/oder Referenz-Farbtemperatur genutzt werden. Zusätzlich wird zumindest ein von einem Nutzer vorzugebender oder vorgegebener Abschatter in einen Bereich der Bilddaten einer Kamera projiziert, um daraus eine erste Helligkeit und/oder erste Farbtemperatur zu bestimmen, um aus Referenz-Helligkeit und einer ersten Helligkeit eine Helligkeitsänderung und/oder aus Referenz-Farbtemperatur und einer ersten Farbtemperatur eine Farbtemperaturänderung zu bestimmen.

## Beschreibung

Hierin werden eine Vorrichtung und ein Verfahren zur Vermessung zumindest einer entlang einer physisch abschatterfreien Messstrecke angeordneten Lichtquelle vorgeschlagen.

Die Qualität einer Beleuchtung einer Lichtquelle, die für die Beleuchtung eines Raumes, einer Oberfläche oder insbesondere eines Operationsfeldes genutzt wird, ist maßgeblich dadurch bestimmt, wie die Helligkeitsverteilung auf der beleuchteten Oberfläche abnimmt, wenn in einen Lichtkegel der Lichtquelle ein Abschatter bewegt wird. Zur Bestimmung einer solchen Qualität einer Beleuchtung werden unter vorgegebenen Situationen genormte Objekte in den Lichtkegel zwischen Lichtaustrittsfläche der Leuchte und der zu beleuchtenden Oberfläche eingebracht und eine durch die eingebrachten Objekte hervorgerufene relative Intensitätsminderung bestimmt.

Aktuell werden für die Bestimmung der relativen Intensitätsminderungen die Abschatter händisch oder robotergestützt in dem Lichtkegel positioniert. Die Messung der Intensität erfolgt über eine Messzelle, die üblicherweise als ein Luxmeter- oder Spektrometerkopf ausgebildet ist. Da für verschiedene Abschatter und/oder Abschatterkonstellationen das Einbringen in den Lichtkegel wiederholt werden muss, ist das aktuelle Vorgehen zur Bestimmung der relativen Intensitätsminderung aufwändig.

Es besteht daher ein Bedarf nach einer Vorrichtung und einem Verfahren zu einer verbesserten und/oder vereinfachten Abschattermessung.

Hierfür werden eine Vorrichtung nach Anspruch 1 und ein Verfahren nach Anspruch 15 vorgeschlagen.

Gemäß einem ersten Aspekt wird eine Vorrichtung zur Bestimmung einer Beleuchtungsänderung einer durch zumindest eine Lichtquelle beleuchteten, insbesondere (physisch) abschatter-freien, Messstrecke vorgeschlagen. Gemäß einem ersten Aspekt kann eine Vorrichtung zur Vermessung zumindest einer entlang einer, insbesondere (physisch) abschatter-freien Messstrecke, angeordneten Lichtquelle vorgeschlagen werden. Die Vorrichtung weist eine (Digital-) Kamera auf, die beispielsweise entlang der Messstrecke, insbesondere in einem, insbesondere lotrechten, ersten Abstand zu der Lichtquelle, angeordnet ist. Die (Digital-) Kamera ist dazu eingerichtet, die zumindest eine Lichtquelle zu erfassen, Bilddaten zu generieren und die Bilddaten, insbesondere an eine Auswerteeinheit, zu senden. Die Vorrichtung weist eine Auswerteeinheit auf. Die Auswerteeinheit ist dazu eingerichtet, die Bilddaten und durch einen Nutzer vorzugebende oder vorgegebene Geometrie- und Relativorts-Daten zumindest eines ersten Abschatters, insbesondere und der zumindest einen Lichtquelle, zu empfangen. Die Auswerteinheit ist dazu eingerichtet, eine Referenz-Helligkeit und/oder Referenz-Farbtemperatur zu berechnen, basierend auf zumindest einem Bereich der Bilddaten, insbesondere und der Geometrie- und Relativorts-Daten. Die Auswerteeinheit ist dazu eingerichtet, zumindest einen ersten Verschattungs-Datensatz zu erstellen, basierend auf dem zumindest einen Bereich der Bilddaten und den vorgegebenen Geometrie- und Relativorts-Daten. Die Auswerteeinheit ist dazu eingerichtet, eine erste, insbesondere reduzierte, Helligkeit zu berechnen, basierend auf dem zumindest einen ersten Verschattungs-Datensatz. Zusätzlich oder alternativ ist die Auswerteeinheit dazu eingerichtet, eine erste Farbtemperatur zu berechnen, basierend auf dem zumindest einen ersten Verschattungs-Datensatz. Die Auswerteeinheit ist dazu eingerichtet, ein Maß für eine erste, insbesondere relative, Helligkeitsänderung zu berechnen, basierend auf der Referenz-Helligkeit und der ersten, insbesondere reduzierten, Helligkeit. Zusätzlich oder alternativ ist die Auswerteeinheit dazu eingerichtet, ein Maß für eine erste, insbesondere relative, Farbtemperaturänderung zu berechnen, basierend auf der Referenz-Farbtemperatur und der ersten Farbtemperatur. Die Auswerteeinheit ist dazu eingerichtet, das Maß für die erste Helligkeitsänderung und/oder das Maß für die erste Farbtemperaturänderung auszugeben.

Der zumindest eine erste Abschatter ist nicht in der Messstrecke vorhanden. Die Messstrecke ist abschatterfrei. Der zumindest eine erste Abschatter kann daher insbesondere als ein projizierter und/oder virtueller und/oder digitaler Abschatter verstanden werden. Der zumindest eine erste Abschatter kann zu einer Intensitätsminderung führen oder beitragen. Andere Objekte als Abschatter können in die Messstrecke eingebracht werden.

Die Vorrichtung gemäß dem ersten Aspekt hat den Vorteil einer vereinfachten Bestimmung/Vermessung. Dies liegt u. a. daran, dass die entsprechenden Abschatter nicht händisch oder robotergestützt für jeden Abschatter und deren Position und Lage erneut in den Lichtkegel eingebracht werden können/sollen/müssen. Ein weiterer Vorteil ist, dass jede einzelne Lichtquelle vermessen werden kann und nicht nur ein Lichtquellentyp. Eine charakteristische Helligkeitsänderung kann somit für alle Lichtquellen eines gleichen Typs individuell bestimmt werden, statt wie herkömmlich die charakteristische Helligkeitsänderung für eine Lichtquelle zu bestimmen und deren charakteristische Helligkeitsänderung für die anderen anzunehmen. Zusätzlich werden systematische Messfehler vermieden, da keine zusätzliche Abschattung durch mechanische Elemente entsteht, die an die Abschatter montiert sind, um die Abschatter in den Lichtkegel einzubringen.

Die Auswerteeinheit kann dazu eingerichtet sein, durch einen Nutzer vorzugebende oder vorgegebene Geometrie- und Relativorts-Daten zumindest eines zweiten Abschatters zu empfangen. Die Auswerteeinheit kann dazu eingerichtet sein, zumindest einen zweiten Verschattungs-Datensatz zu erstellen, basierend auf dem zumindest einen Bereich der Bilddaten und den vorgegebenen Geometrie- und Relativorts-Daten. Die Auswerteeinheit kann dazu eingerichtet sein, eine zweite, insbesondere reduzierte, Helligkeit zu berechnen, basierend auf dem zumindest einen zweiten Verschattungs-Datensatz. Zusätzlich oder alternativ kann die Auswerteeinheit dazu eingerichtet sein, eine zweite Farbtemperatur zu berechnen, basierend auf dem zumindest einen zweiten Verschattungs-Datensatz. Die Auswerteeinheit kann dazu eingerichtet sein, eine Maß für eine zweite, insbesondere relative, Helligkeitsänderung zu berechnen, basierend auf der Referenz-Helligkeit und der zweiten, insbesondere reduzierten, Helligkeit. Zusätzlich oder alternativ kann die Auswerteeinheit dazu eingerichtet sein, eine Maß für eine zweite, insbesondere relative, Farbtemperaturänderung zu berechnen, basierend auf der Referenz-Farbtemperatur und der zweiten Farbtemperatur. Die Auswerteeinheit kann dazu eingerichtet sein, das Maß für die zweite Helligkeitsänderung und/oder das Maß für die zweite Farbtemperaturänderung auszugeben.

Die Auswerteinheit kann dazu eingerichtet sein, zumindest einen weiteren Verschattungs-Datensatz zu erstellen, basierend auf dem zumindest einen Bereich der Bilddaten und den vorgegebenen Geometrie- und Relativorts-Daten (des ersten und zweiten Abschatters). Die Auswerteinheit kann dazu eingerichtet sein, eine weitere, insbesondere reduzierte, Helligkeit zu berechnen, basierend auf dem zumindest einen weiteren Verschattungs-Datensatz. Zusätzlich oder alternativ kann die Auswerteinheit dazu eingerichtet sein, eine weitere Farbtemperatur zu berechnen, basierend auf dem zumindest einen weiteren Verschattungs-Datensatz. Die Auswerteinheit kann dazu eingerichtet sein, eine Maß für eine weitere, insbesondere relative, Helligkeitsänderung zu berechnen, basierend auf der Referenz-Helligkeit und der weiteren, insbesondere reduzierten, Helligkeit. Zusätzlich oder alternativ kann die Auswerteinheit dazu eingerichtet sein, ein Maß für eine weitere, insbesondere relative, Farbtemperaturänderung zu berechnen, basierend auf der Referenz-Farbtemperatur und der weiteren Farbtemperatur. Die Auswerteeinheit kann dazu eingerichtet sein, das Maß für eine/die weitere Helligkeitsänderung und/oder das Maß für eine/die weitere Farbtemperaturänderung auszugeben.

Die Geometriedaten der Lichtquelle und/oder des zumindest einen ersten und/oder des zumindest einen zweiten Abschatters kann/können Information(en) über eine Längs-, Quer- und/oder Hochachsausdehnung, insbesondere entlang einer Längs-, Quer und/oder Hochachse, der Vorrichtung und/oder eines/einer Umrisses/Kontur aufweisen. Der zumindest eine erste und/oder der zumindest eine zweite Abschatter können/kann als eine Vielzahl von Abschattern, als Doppel-Abschatter, als, insbesondere kreisförmiger, Einzelschatter, und/oder als zweifacher, insbesondere zueinander beabstandeter, Einzelschatter ausgebildet sein. Der zumindest eine erste und/oder der zumindest eine zweite Abschatter können/kann als, insbesondere ringförmige, Blende, oder als Objekt mit einer, insbesondere kopfförmigen, handförmigen, körperförmigen oder oberkörperförmigen, Kontur ausgebildet sein. Der zumindest eine erste und/oder der zumindest eine zweite Abschatter können/kann als geometrisches Objekt mit einer Kontur ausgebildet sein, die einer Kontur einer ebenen geometrischen Figur entspricht. Der zumindest eine erste und/oder der zumindest eine zweite Abschatter können/kann als ein Operationswerkzeug ausgebildet sein.

Die Relativorts-Daten können Information(en) über eine Anzahl von Abschattern, insbesondere eine Anzahl der Vielzahl von Abschattern, aufweisen. Die Relativorts-Daten können Information(en) über eine Anzahl von, insbesondere möglichen oder von einem Nutzer vorzugebenden oder vorgegebenen, Abschatter-Konstellationen aus zumindest zwei, drei oder vier oder aus der Vielzahl von Abschattern aufweisen.

Die Auswerteeinheit kann dazu eingerichtet sein, durch einen Nutzer vorzugebende oder vorgegebene Relativorts-Daten des zumindest einen ersten und/oder des zumindest einen zweiten Abschatters und/oder der Vielzahl der Abschattern und der zumindest einen Lichtquelle zu empfangen.

Die Relativorts-Daten können Information(en) aufweisen über eine, insbesondere einzige, Relativlage oder eine Vielzahl von Relativlagen des zumindest einen ersten Abschatters und/oder des zumindest einen zweiten Abschatters insbesondere jeweils der Vielzahl von Abschattern, gegenüber einer Längs-, Quer- und/oder Hochachse, und/oder eines Abschatter- oder Abschatterkonstellationpunkts, insbesondere eines Mittelpunkts oder Drehpunkts.

Die Relativorts-Daten der Lichtquelle und/oder des Abschatters können Information(en) aufweisen über eine, insbesondere einzige, Relativposition oder eine Vielzahl von Relativpositionen, der (Digital-) Kamera gegenüber der Lichtquelle; des zumindest einen ersten und/oder des zumindest zweiten Abschatters gegenüber der Lichtquelle und/oder gegenüber der (Digital-) Kamera; des zumindest einen ersten und/oder des zumindest zweiten Abschatters zueinander und/oder der Vielzahl von Abschattern jeweils zueinander.

Die Relativorts-Daten der Lichtquelle und/oder des Abschatters können Informationen über Lichteigenschaften, insbesondere (graduelle) Opazität, Reflexions- und/oder Transmissionseigenschaften oder Transmissionsspektrum(-spektren), für zumindest einen Teil oder eine (ganze) Oberfläche, des zumindest einen ersten und/oder zweiten Abschatters aufweisen und/oder über eine auf einen Rand des zumindest einen ersten und/oder zweiten Abschatters anzuwendende Gewichtungsfunktion, insbesondere eine während der Erstellung des Verschattungs-Datensatzes anzuwendende Gewichtsfunktion.

Die Auswerteinheit kann dazu eingerichtet sein, eine durch den Nutzer vorzugebende oder vorgegebene Vielzahl von Geometrie- und Relativorts-Daten des zumindest einen ersten und/oder des zumindest einen zweiten Abschatters zu empfangen. Die Auswerteinheit kann dazu eingerichtet sein, eine Vielzahl von Verschattungs-Datensätzen zu erstellen, wobei jeder der Vielzahl von Verschattungs-Datensätzen erstellt wird, basierend auf dem zumindest einen Bereich der Bilddaten und, insbesondere jeweils, einer der Vielzahl von Geometrie- und Relativorts-Daten des ersten und/oder des zweiten Abschatters. Die Auswerteinheit kann dazu eingerichtet sein, eine Vielzahl von, insbesondere reduzierten, Helligkeiten zu berechnen, wobei jede der Vielzahl von, insbesondere reduzierten, Helligkeiten berechnet wird, basierend auf, insbesondere jeweils, einem der Vielzahl der Verschattungs-Datensätze und, insbesondere jeweils, einem der Vielzahl von Geometrie- und Relativorts-Daten. Zusätzlich oder alternativ kann die Auswerteinheit kann dazu eingerichtet sein, eine Vielzahl von Farbtemperaturen zu berechnen, wobei jede der Vielzahl von Farbtemperaturen berechnet wird, basierend auf, insbesondere jeweils, einem der Vielzahl der Verschattungs-Datensätze und, insbesondere jeweils, einem der Vielzahl von Geometrie- und Relativorts-Daten. Die Auswerteinheit kann dazu eingerichtet sein, eine Vielzahl von Maßen für, insbesondere relative, Helligkeitsänderungen zu berechnen und auszugeben, wobei jedes Maß der Vielzahl von Maßen der Helligkeitsänderungen berechnet wird, basierend auf der Referenz-Helligkeit und, insbesondere jeweils, einer der Vielzahl von Helligkeiten oder einer von dem Nutzer vorzugebenden oder vorgegebene Auswahl der Vielzahl von, insbesondere reduzierten, Helligkeiten. Zusätzlich oder alternativ kann die Auswerteinheit dazu eingerichtet sein, eine Vielzahl von Maßen für, insbesondere relative, Farbtemperaturänderungen zu berechnen und auszugeben, wobei jedes Maß der Vielzahl von Maßen für/der Farbtemperaturänderungen berechnet wird, basierend auf der Referenz-Farbtemperatur und, insbesondere jeweils, einer der Vielzahl von Farbtemperaturen oder einer von dem Nutzer vorzugebenden oder vorgegebenen Auswahl der Vielzahl von Farbtemperaturen.

Die Auswerteinheit kann dazu eingerichtet sein, in dem (zu erstellenden) Verschattungs-Datensatz zumindest einen Abschnitt zu bestimmen und dessen Helligkeitswerte zu korrigieren und/oder Farbwerte zu korrigieren, insbesondere zu reduzieren, basierend auf den Bilddaten und den einzugebenden Geometrie- und Relativorts-Daten. Die erste, die zweite, die weitere und/oder die Vielzahl von Helligkeitsänderungen kann/können als Helligkeitsminderung ausgebildet sein. Die erste, die zweite, die weitere oder die Vielzahl Farbtemperaturänderungen können als Farbtemperaturanpassung ausgebildet sein.

Die Auswerteeinheit kann dazu eingerichtet sein, zumindest eine durch den Nutzer vorzugebende oder vorgegebene, insbesondere Mittelwert-, Berechnungsvorschrift für zumindest zwei, drei, vier, fünf oder eine Vielzahl von Verschattungs-Datensätzen zu empfangen. Die Auswerteeinheit kann dazu eingerichtet sein, die erste, zweite, dritte, weitere und/oder Vielzahl von Helligkeiten auf Basis des Ergebnisses der Berechnungsvorschrift zu berechnen. Anders ausgedrückt kann ein Verschattungs-Datensatz berechnet werden, auf Basis der Berechnungsvorschrift und zumindest zwei, drei, vier, fünf oder eine Vielzahl von Verschattungs-Datensätzen. Das Ergebnis der Berechnung mittels einer/der Berechnungsvorschrift kann der Auswerteinheit als Basis für die Berechnung von der ersten, zweiten, dritten, weiteren und/oder der Vielzahl von Helligkeiten dienen.

Die Auswerteeinheit kann dazu eingerichtet sein, zumindest zwei, drei, vier, fünf oder eine Vielzahl von Verschattungs-Datensätzen und die erste, zweite, dritte, weitere und/oder die Vielzahl von Farbtemperaturen auf Basis des Ergebnisses der/einer Berechnungsvorschrift zu berechnen.

Die Lichtquelle kann als eine Kunstlichtquelle oder als eine Leuchte, insbesondere als eine Operationsleuchte, ausgebildet sein.

Der zumindest eine Bereich der Bilddaten kann mit einem Field-of-View (FOV) der (Digital-) Kamera, insbesondere mit einem durch den Nutzer zu wählenden oder gewählten Ausschnitt, des Field-of-Views, korrespondieren.

Der Bereich der Bilddaten kann mit einer, insbesondere gesamten und/oder durch den Nutzer zu wählenden oder gewählten, Lichtaustrittsfläche der zumindest einen Lichtquelle korrespondieren.

Die (Digital-) Kamera kann als Farb-, insbesondere RGB-, Kamera ausgebildet sein. Die (Digital-) Kamera kann als hyperspektrale, insbesondere hyperspektrale 2D-Sensor, Kamera ausgebildet sein. Die (Digital-) Kamera kann als S/W-Kamera ausgebildet sein.

Die eine Auswerteeinheit kann dazu eingerichtet sein, die Bilddaten und durch einen Nutzer vorzugebende oder vorgegebene Geometrie- und Relativorts-Daten zumindest eines ersten Abschatters, insbesondere und der zumindest einen Lichtquelle, zu empfangen. Die Auswerteeinheit kann dazu eingerichtet sein, eine Referenz-Helligkeitsverteilung und/oder eine Referenz-Farbtemperaturverteilung basierend auf zumindest einem Bereich der Bilddaten, insbesondere und der Geometrie- und Relativorts-Daten, zu berechnen. Die Auswerteeinheit kann dazu eingerichtet sein, zumindest einen ersten Verschattungs-Datensatz basierend auf dem zumindest einen Bereich der Bilddaten und den vorgegebenen Geometrie- und Relativorts-Daten zu erstellen. Die Auswerteeinheit kann dazu eingerichtet sein, eine erste Helligkeitsverteilung und/oder eine erste Farbtemperaturverteilung, insbesondere entlang des Bereiches, basierend auf dem zumindest einen ersten Verschattungs-Datensatz zu berechnen. Die Auswerteeinheit kann dazu eingerichtet sein, ein zumindest, insbesondere auf (zumindest) ein Pixel der Kamera bezogenes, ortsaufgelöstes Maß für eine erste Helligkeitsänderung basierend auf der Referenz-Helligkeitsverteilung und der ersten Helligkeitsverteilung zu berechnen und/oder ein zumindest, insbesondere auf das oder (zumindest) ein (weiteres) Pixel der Kamera bezogenes, ortsaufgelöstes Maß für eine erste Farbtemperaturänderung basierend auf der Referenz-Farbtemperaturverteilung und der ersten Farbtemperaturverteilung zu berechnen. Die Auswerteeinheit kann dazu eingerichtet sein, das Maß für die erste Helligkeitsänderung und/oder das Maß für die erste Farbtemperaturänderung auszugeben.

Dies hat den Vorteil, dass Farbtemperaturen und Helligkeitswerte für jedes oder pro Pixel genau bestimmt werden können. Im Falle einer hyperspektralen Kamera kann für jedes Pixel das durch den simulierten Abschatter geänderte Spektrum simuliert und damit die Farbtemperatur bestimmt werden, die sich für eine reale Abschattung ergeben würde. Damit kann simulativ pixelgenau bestimmt werden, wie sich die Farbtemperatur an der durch die Lichtquelle beleuchteten Oberfläche ändert/einstellt. Hinsichtlich der Anwendung auf chirurgischem Gebiet hat dies den Vorteil, dass abgeschätzt werden kann, wie gut die Lichtquelle das Operationsfeld (z. B. einen Bereich auf der Haut) wiedergibt. Bei der Beleuchtung von Objekten mittels der Leuchte kann deren Farbwirkungsverteilung am Objekt vor der praktischen Umsetzung für verschiedene Abschattungsszenarien simuliert werden. Denn die Farbtemperatur und/oder Helligkeitsverteilung kann entlang der Lichtaustrittsfläche pixelgenau aufgelöst werden.

Die Auswerteeinheit kann dazu eingerichtet sein, Linsenabbildungsfehler der Kamera und/oder perspektivische Verzerrungen zu korrigieren, insbesondere in den Bilddaten.

Die Auswerteeinheit kann dazu eingerichtet sein, der zumindest einen Lichtquelle den Verschattungsdatensatz zu senden, auf Basis dessen die Lichtquelle ein Schattenmanagement durchführt, insbesondere einzelne Bereiche der Lichtaustrittsfläche in deren Intensität reduziert oder erhöht. Die (Digital-) Kamera kann dazu eingerichtet sein, die im Schattenmanagement befindliche zumindest eine Lichtquelle zu erfassen, insbesondere zu jeweils unterschiedlichen Zeitpunkten/über zumindest einen Zeitabschnitt eine Vielzahl von Bilddaten zu generieren und die Bilddaten, insbesondere an eine Auswerteeinheit, zu senden. Die Auswerteeinheit kann dazu eingerichtet, Bilddaten der zumindest einen im Schattenmanagement befindlichen Lichtquelle zu empfangen. Die Auswerteinheit ist dazu eingerichtet, eine resultierende Helligkeit und/oder resultierende Farbtemperatur zu berechnen, basierend auf den Bilddaten, insbesondere jeweils eine der Vielzahl von Bilddaten.

Vorteil ist, dass die Wirkung des Schattenmanagements beurteilt werden kann, ohne eine physische Abschattung der Lichtquelle. Anders ausgedrückt kann geprüft werden, welche Messwerte bei aktivem Schatten-Management der Lichtquelle erreicht werden können.

Gemäß einem zweiten Aspekt wird ein Verfahren zur, insbesondere simulativen, Bestimmung einer Beleuchtungsänderung einer durch zumindest eine Lichtquelle beleuchteten, insbesondere (physisch) abschatter-freien, Messstrecke vorgeschlagen.

Gemäß einem zweiten Aspekt kann ein Verfahren zur Vermessung zumindest einer entlang einer, insbesondere (physisch) abschatter-freien, Messstrecke angeordneten Lichtquelle vorgeschlagen werden.

Das Verfahren umfass ein Bereitstellen einer (Digital-) Kamera, die entlang der Messstrecke angeordnet ist. Die (Digital-) Kamera kann entlang der Messstrecke in einem, insbesondere lotrechten, ersten Abstand zu der Lichtquelle, angeordnet sein. Die (Digital-) Kamera ist dazu eingerichtet, die zumindest eine Lichtquelle zu erfassen. Das Verfahren umfasst ein Generieren von Bilddaten, insbesondere mittels der (Digital-) Kamera. Das Verfahren umfasst ein Bereitstellen einer Auswerteeinheit. Das Verfahren umfasst ein Senden der Bilddaten, insbesondere an eine/die Auswerteeinheit, beispielsweise mittels der (Digital-) Kamera. Das Verfahren umfasst ein Empfangen, mittels der Auswerteeinheit, der Bilddaten und von durch einen Nutzer vorzugebenden oder vorgegebenen Geometrie- und Relativorts-Daten zumindest eines ersten Abschatters, insbesondere und der zumindest einen Lichtquelle. Das Verfahren umfasst ein Berechnen, mittels der Auswerteeinheit, einer Referenz-Helligkeit und/oder Referenz-Farbtemperatur basierend auf zumindest einem Bereich der Bilddaten, insbesondere und der Geometrie- und Relativortsdaten. Das Verfahren umfasst ein Erstellen, mittels der Auswerteeinheit, zumindest eines ersten Verschattungs-Datensatzes, basierend auf dem zumindest einen Bereich der Bilddaten und den vorgegebenen Geometrie- und Relativorts-Daten. Das Verfahren umfasst ein Berechnen, mittels der Auswerteeinheit, einer ersten, insbesondere reduzierten, Helligkeit und/oder ersten Farbtemperatur, basierend auf dem zumindest einen ersten Verschattungs-Datensatz. Das Verfahren umfasst ein Berechnen, mittels der Auswerteeinheit, eines Maßes für eine erste Helligkeitsänderung, basierend auf der Referenz-Helligkeit und der ersten, insbesondere reduzierten, Helligkeit. Zusätzlich oder alternativ umfasst das Verfahren ein Berechnen, mittels der Auswerteeinheit, eines Maßes für eine erste Farbtemperaturänderung, basierend auf der Referenz-Farbtemperatur und der ersten Farbtemperatur. Das Verfahren umfasst ein Ausgeben, mittels der Auswerteeinheit, des Maßes für die erste Helligkeitsänderung und/oder des Maßes der ersten Farbtemperaturänderung.

Weitere Merkmale, Eigenschaften, Vorteile und mögliche Abwandlungen werden für einen Fachmann anhand der nachstehenden Beschreibungen deutlich, in der auf die beigefügten Zeichnungen Bezug genommen ist.
- Fig.1: zeigt eine schematische Darstellung des Standes der Technik.
- Fig.2: zeigt eine Variante einer Ausführungsform der Vorrichtung mit einem projizierten Abschatter.
- Fig.3: zeigt eine weitere Variante einer Ausführungsform der Vorrichtung mit zwei projizierten Abschattern und einer sich daraus ergebenden Schatter-Konstellation.
- Fig.4: zeigt eine weitere Variante einer Ausführungsform der Vorrichtung mit einem projizierten zweifachen Einzelschatter in verschiedenen Relativlagen.
- Fig.5: zeigt eine schematische Darstellung der Vorrichtung mit drei projizierten Abschattern und mehreren Schatter-Konstellationen.
- Fig.6: zeigt eine schematische Darstellung der Vorrichtung zur Anwendung in einem Raum.
- Fig.7: zeigt ein Flussdiagramm eines Verfahrens zur Vermessung zumindest einer entlang einer physisch abschatter-freien Messstrecke angeordneten Lichtquelle.

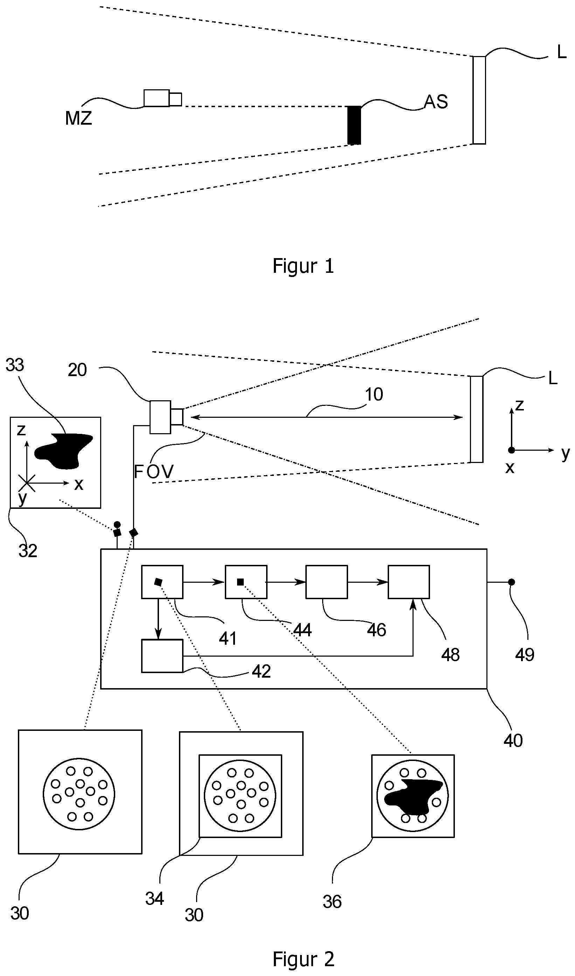

Fig.1 zeigt eine schematische Darstellung des Standes der Technik. Dargestellt ist eine Messzelle MZ, die als einzelner Luxmeterkopf ausgebildet ist. Der Luxmeterkopf misst den integralen Referenz-Helligkeitswert der Leuchte L, bevor der Abschatter AS in den Lichtkegel eingebracht wird (diese Variante ist nicht abgebildet). Für eine Messung eines reduzierten Helligkeitswerts wird ein Abschatter AS in den Lichtkegel der Leuchte L wie abgebildet eingebracht. Aufgrund der Abschattung misst der Luxmeterkopf einen reduzierten integralen Helligkeitswert. Aus dem Referenz-Helligkeitswert und dem reduzierten Helligkeitswert wird im Stand der Technik eine relative Helligkeitsminderung berechnet, die Rückschlüsse auf die Beleuchtungsqualität der Leuchte während einer Abschattung zulässt. Üblicherweise werden nicht nur eine einzelne Relativposition und Relativlage des Abschatters zu Berechnung der relativen Helligkeitsminderung herangezogen, sondern mehrere Relativpositionen und/oder Relativlagen. Dies führt zu langwierigen und aufwendigen Messverfahren.

Fig.2 zeigt eine Variante einer Ausführungsform der Vorrichtung mit einem einzigen projizierten Abschatter. Die Vorrichtung weist eine Kamera 20 auf, die in der dargestellten Variante als Digital-Kamera ausgebildet ist und ein Field-Of-View FOV aufweist. In dem Field-Of-View ist in einem ersten Abstand 10 zur Digital-Kamera 20, entlang einer Längsachse y der Vorrichtung, eine Lichtquelle L angeordnet, die ein Licht in Richtung der Digital-Kamera 20 abgibt. Die Digital-Kamera 20 ist dazu eingerichtet, die Lichtquelle L zu erfassen, Bilddaten 30 zu generieren und die Bilddaten 30 an eine Auswerteeinheit 40 zu senden. Die mit dem Bezugszeichen 30 bezeichneten Bilddaten zeigen die von der Digital-Kamera 20 erfasste Lichtquelle L. Zu erkennen ist, dass die Bilddaten 30 mit dem Field-of-View der Digital-Kamera 20 korrespondieren. Die Lichtquelle L selbst weist in der dargestellten Variante eine Vielzahl von Lichtelementen auf.

Zusätzlich zu den Bilddaten 30 ist die Auswerteeinheit 40 dazu eingerichtet, Geometrie- und Relativorts-Daten 32 eines Abschatters 33 und der Lichtquelle L zu empfangen. In der dargestellten Variante weisen die Geometrie- und Relativorts-Daten 32 eine Information über die Kontur des Abschatters 33 sowie über dessen relative Position und dessen relative Lage in Bezug zu einer Längs-, Quer- und Hochachse y, x, z der Vorrichtung auf. Die Information über Relativpostion und Relativlage der Lichtquelle L in Bezug zu der Längs-, Quer- und Hochachse y, x, z ist nicht grafisch in den Geometrie- und Relativorts-Daten 32 dargestellt.

Mit anderen Worten gibt der Nutzer vor, für welche Geometrie- und Relativorts-Daten 32 der Abschatter 33 in die Bilddaten 30 projiziert werden soll. Hat der Nutzer diese Informationen vorgegeben, erfolgt die weitere Verarbeitung der Bilddaten 30 und der Geometrie- und Relativorts-Daten 32 in der Auswerteeinheit 40.

Die dargestellte Auswerteeinheit 40 weist fünf Blöcke 41, 42, 44, 46, 48 auf, die die Verarbeitung der Geometrie- und Relativorts-Daten 32 und der Bilddaten 30 in der Auswerteeinheit 40 verdeutlichen sollen.

Zunächst wird in Block 41 ein Bereich 34 der Bilddaten 30 ausgewählt. Zusätzlich kann vor oder nach der Bereichsauswahl eine Umrechnung des Bereich 34 der Bilddaten 30 erfolgen, um die später zu berechnende Referenz-Helligkeit oder erste Helligkeit zu berechnen, bspw. von RGB zu Lab. Der Bereich 34 kann entweder durch den Nutzer selbst in den Bilddaten 30 ausgewählt werden, oder die Auswerteeinheit 40 kann auf Basis der Geometrie- und Relativorts-Daten 32 den Bereich 34 erkennen und jenen entsprechend auswählen. Dieser Bereich 34 dient als Grundlage für die zu berechnende Referenz-Helligkeit und der ersten Helligkeit.

Die Referenz-Helligkeit wird anhand des Bereichs 34 ermittelt (Block 42), indem über alle Pixel-Werte des Bereichs 34 integriert wird. Die in Block 42 berechnete Referenz-Helligkeit dient als Grundlage für eine Berechnung eines Maßes einer ersten Helligkeitsänderung.

Für die Berechnung der ersten Helligkeit wird ein erster Verschattungs-Datensatz 36 erstellt (Block 44). Dafür werden im vorliegenden Beispiel die Pixel-Werte des Bereichs 34 genullt oder mit dem Wert Null ersetzt, die gemäß durch den Nutzer vorgegebenen Geometrie- und Relativorts-Daten 32 einer Fläche des Abschatters 33 entsprechen. Auf Basis dieses Verschattungs-Datensatzes 36 wird eine erste Helligkeit berechnet (Block 46), indem über alle Pixel-Werte des Verschattungs-Datensatzes 36 integriert wird.

Letztlich wird das Maß der ersten Helligkeitsänderung berechnet (Block 48) indem aus der ersten Helligkeit und der Referenz-Helligkeit ein Quotient berechnet und jener als Maß der ersten Helligkeit ausgegeben wird (siehe 49).

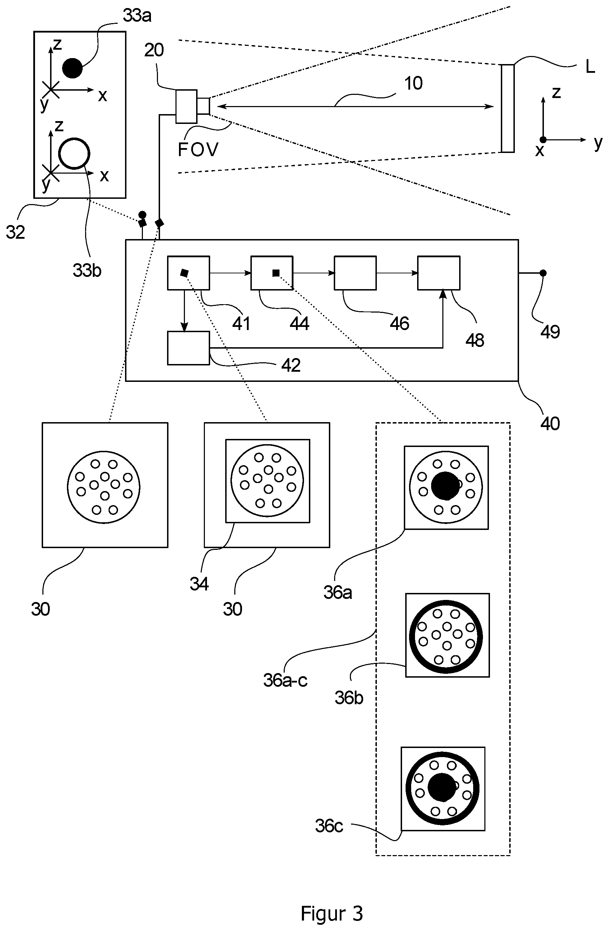

Fig.3 zeigt eine weitere Variante einer Ausführungsform der Vorrichtung mit zwei projizierten Abschattern/Schattern und einer projizierten Schatter-Konstellation. Die Vorrichtung weist eine Kamera 20 auf, die in der dargestellten Variante als Digital-Kamera 20 ausgebildet ist und ein Field-Of-View FOV aufweist. In dem Field-Of-View FOV ist in einem ersten Abstand 10 zur Digital-Kamera 20, entlang einer Längsachse y der Vorrichtung, eine Lichtquelle L angeordnet, die ein Licht in Richtung der Digital-Kamera 20 abgibt. Die Digital-Kamera 20 ist dazu eingerichtet, die Lichtquelle L zu erfassen, Bilddaten 30 zu generieren und die Bilddaten 30 an eine Auswerteeinheit 40 zu senden. Die mit dem Bezugszeichen 30 bezeichneten Bilddaten zeigen die von der Digital-Kamera 20 erfasste Lichtquelle L. Zu erkennen ist, dass die Bilddaten 30 mit dem Field-of-View der Digital-Kamera 20 korrespondieren. Die Lichtquelle L selbst weist in der dargestellten Variante eine Vielzahl von Lichtelementen auf.

Zusätzlich zu den Bilddaten 30 ist die Auswerteeinheit 40 dazu eingerichtet, Geometrie- und Relativorts-Daten 32 eines ersten Abschatters 33a, eines zweiten Abschatters 33b und der Lichtquelle L zu empfangen. In der dargestellten Variante ist der erste Abschatter 33a als kreisförmiger Einzelschatter und der zweite Abschatter 33b als ringförmige Blende ausgebildet.

In der dargestellten Variante weisen die Geometrie- und Relativorts-Daten 32 eine Information über eine Kontur des kreisförmigen Einzelschatters 33a und eine Kontur der ringförmigen Blende 33b auf, sowie über deren Relativposition und deren Relativlage in Bezug zu einer Längs-, Quer-, und Hochachse y, x, z der Vorrichtung. Zusätzlich weisen die Geometrie- und Relativorts-Daten 32 die mögliche Anzahl an Schatter-Konstellationen auf. In der dargestellten Variante ergibt sich eine einzige mögliche Schatter-Konstellation zwischen dem kreisförmigen Einzelschatters 33a und der ringförmigen Blende 33b.

Die Information über Relativposition und Relativlage der Lichtquelle L in Bezug zu der Längs-, Quer-, und Hochachse y, x, z ist nicht grafisch in den Geometrie- und Relativorts-Daten 32 dargestellt.

Der Nutzer gibt vor, für welche Geometrie- und Relativorts-Daten 32 der kreisförmigen Einzelschatters 33a, der ringförmigen Blende 33b und welche Schatter-Konstellation in die Bilddaten 30 projiziert werden soll. Hat der Nutzer diese Informationen vorgegeben, erfolgt die weitere Verarbeitung der Bilddaten 30 und der Geometrie- und Relativorts-Daten 32 in der Auswerteeinheit 40.

Die dargestellte Auswerteeinheit 40 weist fünf Blöcke 41, 42, 44, 46, 48 auf, die die Verarbeitung der Geometrie- und Relativorts-Daten 32 und der Bilddaten 30 in der Auswerteeinheit 40 verdeutlichen sollen.

Zunächst wird ein Bereich 34 der Bilddaten 30 ausgewählt 41. Zusätzlich kann vor oder nach der Bereichsauswahl eine Umrechnung des Bereich oder der Bilddaten erfolgen, um die später zu berechnende Referenz-Helligkeit oder erste Helligkeit vereinfacht zu berechnen, bspw. von RGB zu Lab. Der Bereich 34 kann entweder durch den Nutzer selbst in den Bilddaten 30 ausgewählt werden oder die Auswerteeinheit 40 kann dazu eingerichtet sein, auf Basis der Geometrie- und Relativorts-Daten 32 den Bereich 34 zu erkennen und jenen entsprechend auszuwählen bspw. mit Methoden des Image Processings. Der Bereich 34 dient als Grundlage für die zu berechnende Referenz-Helligkeit sowie für die zu berechnende erste, zweite und weitere Helligkeit.

Die Referenz-Helligkeit wird anhand des Bereichs 34 ermittelt (Block 42), indem über alle Pixel-Werte des Bereichs 34 integriert wird. Die in Block 42 berechnete Referenz-Helligkeit dient als Grundlage für eine Berechnung jeweils eines Maßes für eine erste, zweite und weitere Helligkeitsänderung.

Für die Berechnung der ersten Helligkeit wird ein erster Verschattungs-Datensatz 36a erstellt 44. Dafür werden im vorliegenden Beispiel die Pixel-Werte des Bereich 34 genullt oder mit dem Wert Null ersetzt, die gemäß durch den Nutzer vorgegebenen Geometrie- und Relativorts-Daten 32 einer Fläche des kreisförmigen Einzelschatters 33a entsprechen. Auf Basis dieses ersten Verschattungs-Datensatzes 36a wird eine erste Helligkeit berechnet 46, indem über alle Pixel-Werte des Verschattungs-Datensatzes 36a integriert wird.

Für die Berechnung einer zweiten Helligkeit wird ein zweiter Verschattungs-Datensatz 36b erstellt 44. Dafür werden im vorliegenden Beispiel die Pixel-Werte des Bereichs 34 genullt oder mit dem Wert Null ersetzt, die gemäß durch den Nutzer vorgegebenen Geometrie- und Relativorts-Daten 32 einer Fläche der ringförmigen Blende 33b entsprechen. Auf Basis dieses zweiten Verschattungs-Datensatzes 36b wird eine zweite Helligkeit berechnet 46, indem über alle Pixel-Werte des Verschattungs-Datensatzes 36b integriert wird.

Für die Berechnung einer weiteren Helligkeit wird ein weiterer Verschattungs-Datensatz 36c erstellt (Block 44). Dafür werden im vorliegenden Beispiel die Pixel-Werte des Bereichs 34 genullt oder mit dem Wert Null ersetzt, die gemäß durch den Nutzer vorgegebenen Geometrie- und Relativorts-Daten 32 einer Fläche des kreisförmigen Einzelschatters 33a und der ringförmigen Blende 33b entsprechen. Auf Basis dieses weiteren Verschattungs-Datensatzes 36c wird eine weitere Helligkeit berechnet (Block 46), indem über alle Pixel-Werte des weiteren Verschattungs-Datensatzes 36b integriert wird.

Letztlich wird ein Maß einer ersten, zweiten und weiteren Helligkeitsänderung berechnet (Block 48).

Das Maß für die erste Helligkeitsänderung wird auf Basis der berechneten ersten Helligkeit und der Referenz-Helligkeit berechnet (Block 48) und ausgegeben (Block 49). Das Maß für die zweite Helligkeitsänderung wird auf Basis der berechneten zweiten Helligkeit und der Referenz-Helligkeit berechnet (Block 48) und ausgegeben (Block 49). Das Maß für die weitere Helligkeitsänderung wird entsprechend auf Basis der berechneten weiteren Helligkeit und der Referenz-Helligkeit berechnet (Block 48) und ausgegeben (Block 49).

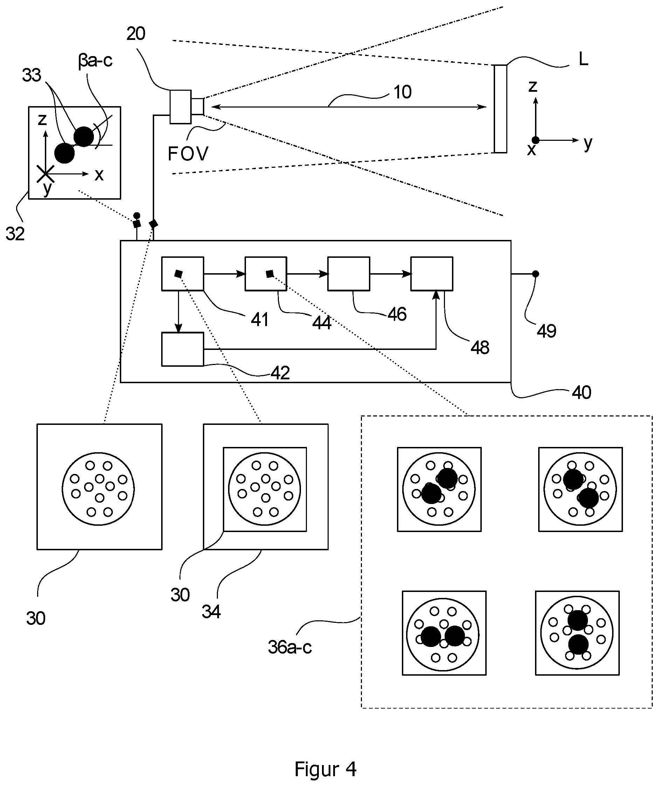

Fig.4 zeigt eine weitere Variante einer Ausführungsform der Vorrichtung mit einem projizierten zweifachen Einzelschatter in verschiedenen Relativlagen. Die Vorrichtung weist eine Kamera 20 auf, die in der dargestellten Variante als Digital-Kamera 20 ausgebildet ist und ein Field-Of-View FOV aufweist. In dem Field-Of-View FOV ist in einem ersten Abstand 10 zur Digital-Kamera 20, entlang einer Längsachse y der Vorrichtung, eine Lichtquelle L angeordnet, die ein Licht in Richtung der Digital-Kamera 20 abgibt. Die Digital-Kamera 20 ist dazu eingerichtet, die Lichtquelle L zu erfassen, Bilddaten 30 zu generieren und die Bilddaten 30 an eine Auswerteeinheit 40 zu senden. Die mit dem Bezugszeichen 30 bezeichneten Bilddaten zeigen die von der Digital-Kamera 20 erfasste Lichtquelle L. Zu erkennen ist, dass die Bilddaten 30 mit dem Field-of-View FOV der Digital-Kamera 20 korrespondieren. Die Lichtquelle L selbst weist in der dargestellten Variante eine Vielzahl von Lichtelementen auf. In der dargestellten Variante ist die Lichtquelle als kreisförmige Operationsleuchte ausgebildet.

Zusätzlich zu den Bilddaten 30 ist die Auswerteeinheit 40 dazu eingerichtet, Geometrie- und Relativorts-Daten 32 eines zweifachen Einzelschatters 33 und der Lichtquelle L zu empfangen. In der dargestellten Variante weisen die Geometrie- und Relativorts-Daten 32 eine Information über die Kontur des zweifachen Einzelschatters 33, sowie über dessen Relativposition in Bezug zu einer Längs-, Quer- und Hochachse y, x, z der Vorrichtung auf, sowie eine Vielzahl von Relativlagen in Form eines Drehwinkel β um einen gemeinsamen Drehpunkt des zweifachen Einzelschatters 33.

Die Information über Relativposition und relative Lage der Lichtquelle L in Bezug zu der Längs-, Quer- und Hochachse y, x, z ist nicht grafisch in den Geometrie- und Relativorts-Daten 32 dargestellt.

Der Nutzer gibt vor, für welche Geometrie- und Relativorts-Daten 32 der zweifache Einzelschatter 33 in die Bilddaten 30 projiziert werden soll. Hat der Nutzer diese Informationen vorgegeben, erfolgt die weitere Verarbeitung der Bilddaten 30 und der Geometrie- und Relativorts-Daten 32 in der Auswerteeinheit 40.

In der dargestellten Variante hat der Nutzer vorgegeben, dass ein Abschatter in Form eines zweifachen Einzelschatters 33 projiziert werden soll, dessen Drehpunkt mit einem Zentrum der Operationsleuchte korrespondiert. Zusätzlich wurde durch den Nutzer vorgegeben, dass für vier verschiedene Drehwinkel βa-c der zweifache Einzelschatter 33 in die Bilddaten projiziert werden soll.

Der Nutzer gibt der Auswerteeinheit 40 weiter vor, wie die Verschattungs-Datensätze mittels einer Berechnungsvorschrift miteinander verrechnet werden sollen, bevor eine erste Helligkeit berechnet wird.

Die dargestellte Auswerteeinheit 40 weist fünf Blöcke 41, 42, 44, 46, 48 auf, welche die Verarbeitung der Geometrie- und Relativorts-Daten 32 und der Bilddaten 30 in der Auswerteeinheit 40 verdeutlichen sollen.

Zunächst wird ein Bereich 34 der Bilddaten 30 ausgewählt (Block 41). Zusätzlich kann vor oder nach der Bereichsauswahl eine Umrechnung des Bereich oder der Bilddaten erfolgen, um die später zu berechnende Referenz-Helligkeit oder erste Helligkeit vereinfacht zu berechnen, bspw. von RGB zu Lab. Dieser Bereich 34 kann entweder durch den Nutzer selbst in den Bilddaten 30 ausgewählt werden. Zusätzlich oder alternativ kann die Auswerteeinheit 40 dazu eingerichtet sein, auf Basis der Geometrie- und Relativorts-Daten 32 den Bereich 34 zu erkennen und jenen entsprechend auszuwählen bspw. mittels Methoden des Image Processing. Dieser Bereich 34 dient als Grundlage für die zu berechnende Referenz-Helligkeit und der ersten Helligkeit.

Die Referenz-Helligkeit wird anhand des Bereichs 34 ermittelt (Block 42), indem über alle Pixel-Werte des Bereichs 34 integriert wird. Die in Block 42 berechnete Referenz-Helligkeit dient als Grundlage für eine Berechnung der ersten Helligkeitsänderung.

Für die Berechnung der ersten Helligkeit werden vier Verschattungs-Datensätze 36a-c erstellt (Block 44). Dafür werden im vorliegenden Beispiel die Pixel-Werte des Bereich 34 jeweils genullt oder mit dem Wert Null ersetzt, die gemäß durch den Nutzer vorgegebenen Geometrie- und Relativorts-Daten 32 einer Fläche des zweifachen Einzelschatters 33 entsprechen. Anders ausgedrückt entspricht jeder Verschattungs-Datensatz 36a-c einer der Projektion des zweifacheb Einzelschatters für einen der durch den Nutzer vorgegebenen Drehwinkel βa-c in den Bereich 34 der Bilddaten und damit in die Abbildung der Lichtquelle.

Aus den vier Verschattungs-Datensätzen 36a-c wird ein gemittelter Verschattungs-Datensatz berechnet, basierend auf der Berechnungsvorschrift. Aus dem gemittelten Verschattungs-Datensatz wird die erste Helligkeit berechnet, indem über alle Pixel-Werte des gemittelten Verschattungs-Datensatz integriert wird.

Letztlich wird ein Maß einer erste Helligkeitsänderung berechnet (Block 48) und zwar auf Basis der berechneten ersten Helligkeit und der Referenz-Helligkeit und jenes Maß wird ausgegeben (Block 49).

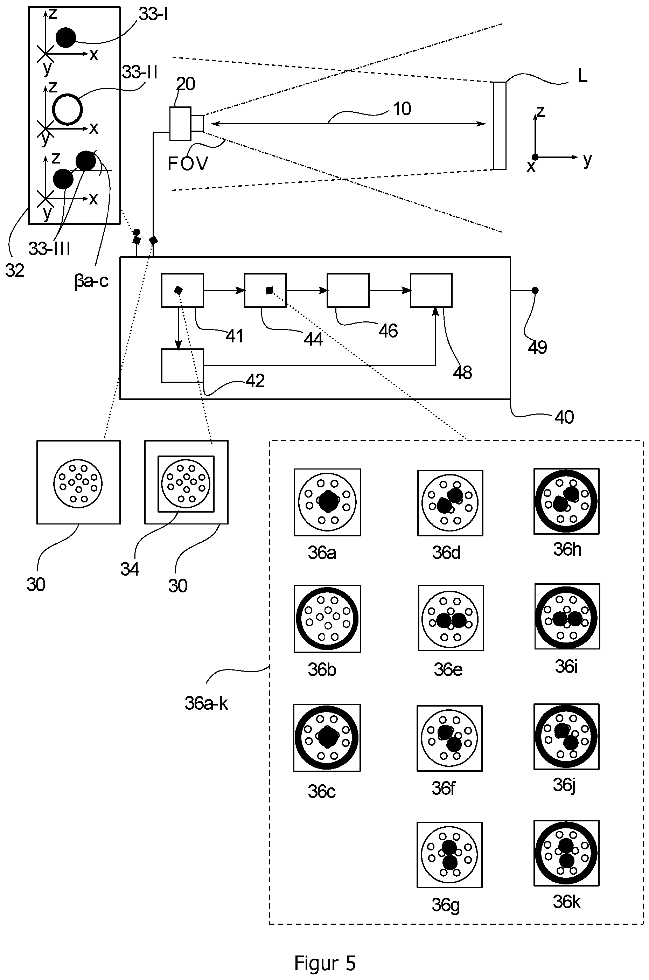

Fig.5 zeigt eine schematische Darstellung der Vorrichtung mit drei projizierten Abschattern 33-I, 33-II, 33-III und eine Auswahl an projizierten Schatter-Konstellationen. Die Vorrichtung weist eine Kamera 20 auf, die in der dargestellten Variante als Digital-Kamera 20 ausgebildet ist und ein Field-Of-View FOV aufweist. In dem Field-Of-View FOV ist in einem ersten Abstand 10 zur Digital-Kamera 20, entlang einer Längsachse y der Vorrichtung, eine Lichtquelle L angeordnet, die ein Licht in Richtung der Digital-Kamera 20 abgibt. Die Digital-Kamera 20 ist dazu eingerichtet, die Lichtquelle L zu erfassen, Bilddaten 30 zu generieren und die Bilddaten 30 an eine Auswerteeinheit 40 zu senden. Die mit dem Bezugszeichen 30 bezeichneten Bilddaten zeigen die von der Digital-Kamera 20 erfasste Lichtquelle L. Zu erkennen ist, dass die Bilddaten 30 mit dem Field-of-View FOV der Digital-Kamera 20 korrespondieren. Die Lichtquelle L ist in der dargestellten Variante als Operationsleuchte ausgebildet und weist eine Vielzahl von Lichtelementen auf.

Zusätzlich zu den Bilddaten 30 ist die Auswerteeinheit 40 dazu eingerichtet, Geometrie- und Relativorts-Daten 32 eines ersten Abschatters 33-I, eines zweiten Abschatters 33-II und eines dritten Abschatters 33-III der Lichtquelle L zu empfangen. In der dargestellten Variante ist der erste Abschatter 33-I als kreisförmiger Einzelschatter, der zweite Abschatter 33-II als ringförmige Blende und der dritte Abschatter 33-III als zweifacher Einzelschatter ausgebildet.

In der dargestellten Variante weisen die Geometrie- und Relativorts-Daten 32 eine Information über die Kontur des kreisförmigen Einzelschatters 33-I, der ringförmigen Blende 33-II und des zweifachen Einzelschatters 33-III auf, sowie über deren Relativposition in Bezug zu einer Längs-, Quer-, und Hochachse y, x, z der Vorrichtung. Zusätzlich weisen die Geometrie- und Relativorts-Daten 32 eine Vielzahl von Relativlagen des zweifachen Einzelschatters 33-III auf und zwar in Form eines Drehwinkels βa-c um einen gemeinsamen Drehpunkt des zweifachen Einzelschatters 33-III auf.

Zusätzlich weisen die Geometrie- und Relativorts-Daten 32 die vom Nutzer vorgegebene Anzahl an Schatter-Konstellationen auf. In der dargestellten Variante ergibt sich eine durch den Nutzer vorgegebene Schatter-Konstellation zwischen dem kreisförmigen Einzelschatters 33-I und der ringförmigen Blende 33-II, sowie zwischen der ringförmigen Blende 33-II und dem zweifachen Einzelschatter 33-III.

Die Information über Relativpostion und Relativlage die Lichtquelle L in Bezug zu der Längs-, Quer- und Hochachse y, x, z ist nicht grafisch in den Geometrie- und Relativorts-Daten 32 dargestellt.

Der Nutzer gibt vor, für welche Geometrie- und Relativorts-Daten 32 der kreisförmigen Einzelschatter 33-I, die ringförmige Blende 33-II und die Schatter-Konstellation in den Bereich 34 der Bilddaten 30 projiziert werden soll. In der vorliegenden Variante gibt der Nutzer vor, dass der zweifache Einzelschatter 33-III und die Schatterkonstellation zwischen dem zweifachen Einzelschatters 33-III und der ringförmigen Blende 33-II für jeden Drehwinkel βa-c in den Bereich 34 projiziert werden sollen.

Des Weiteren gibt der Nutzer vor, welche Verschattungs-Datensätze zur Berechnung einer ersten bis fünften Helligkeitsänderung genutzt werden sollen und wie die Auswahl an Verschattungs-Datensätzen mittels einer Berechnungsvorschrift miteinander verrechnet werden sollen. In der vorliegenden Variante sollen die Verschattungsdatensätze 36d-g des zweifachen Einzelschatters 33-III gemittelt werden, bevor eine Helligkeit berechnet wird. Auch die Verschattungs-Datensätzen 36h-36k der Schatter-Konstellation zwischen dem zweifachen Einzelschatter 33-III und der ringförmiger Blende 33-II sollen gemittelt werden.

Hat der Nutzer diese Informationen vorgegeben, erfolgt die weitere Verarbeitung der Bilddaten 30 und der Geometrie- und Relativorts-Daten 32 in der Auswerteeinheit 40.

Die dargestellte Auswerteeinheit 40 weist fünf Blöcke 41, 42, 44, 46, 48 auf, welche die Verarbeitung der Geometrie- und Relativorts-Daten 32 und der Bilddaten 30 in der Auswerteeinheit 40 verdeutlichen sollen.

Zunächst wird ein Bereich 34 der Bilddaten 30 ausgewählt 41. Zusätzlich kann vor oder nach der Bereichsauswahl eine Umrechnung des Bereichs oder der Bilddaten erfolgen, um die später zu berechnende Referenz-Helligkeit oder erste Helligkeit vereinfacht zu berechnen, bspw. von RGB zu Lab. Dieser Bereich 34 kann entweder durch den Nutzer selbst in den Bilddaten 30 ausgewählt werden. Alternativ kann die Auswerteeinheit 40 dazu eingerichtet sein, bswp. mittels einer Methode des Image Processings auf Basis der Geometrie- und Relativorts-Daten 32 den Bereich 34 zu erkennen und jenen entsprechend auszuwählen. Dieser Bereich 34 dient als Grundlage für die zu berechnende Referenz-Helligkeit und der ersten bis fünften Helligkeit.

Die Referenz-Helligkeit wird anhand des Bereichs 34 berechnet (Block 42), indem über alle Pixel-Werte des Bereichs 34 integriert wird. Die in Block42 berechnete Referenz-Helligkeit dient als Grundlage für eine Berechnung der Helligkeitsänderung.

Für die Berechnung der ersten Helligkeit wird ein erster Verschattungs-Datensatz 36a erstellt (Block 44). Dafür werden im vorliegenden Beispiel die Pixel-Werte des Bereich 34 genullt oder mit dem Wert Null ersetzt, die gemäß durch den Nutzer vorgegebenen Geometrie- und Relativorts-Daten 32 einer Fläche des kreisförmigen Einzelschatters 33-I entsprechen. Auf Basis dieses ersten Verschattungs-Datensatzes 36a wird eine erste Helligkeit berechnet 46, indem über alle Pixel-Werte des Verschattungs-Datensatzes 36a integriert wird.

Für die Berechnung einer zweiten Helligkeit wird ein zweiter Verschattungs-Datensatz 36b erstellt 44. Dafür werden im vorliegenden Beispiel die Pixel-Werte des Bereich 34 genullt oder mit dem Wert Null ersetzt, die gemäß durch den Nutzer vorgegebenen Geometrie- und Relativorts-Daten 32 einer Fläche der ringförmigen Blende 33-II entsprechen. Auf Basis dieses zweiten Verschattungs-Datensatzes 36b wird eine zweite Helligkeit berechnet (Block 46), indem über alle Pixel-Werte des Verschattungs-Datensatzes 36b integriert wird.

Für die Berechnung einer dritten Helligkeit wird ein dritter Verschattungs-Datensatz 36c erstellt (Block 44). Dafür werden im vorliegenden Beispiel die Pixel-Werte des Bereich 34 genullt oder mit dem Wert Null ersetzt, die gemäß durch den Nutzer vorgegebenen Geometrie- und Relativorts-Daten 32 einer Fläche des kreisförmigen Einzelschatters 33-I und der ringförmigen Blende 33-II entsprechen. Auf Basis dieses dritten Verschattungs-Datensatzes 36c wird eine dritte Helligkeit berechnet (Block 46), indem über alle Pixel-Werte des Verschattungs-Datensatzes 36c integriert wird.

Für die Berechnung einer vierten Helligkeit, werden vier weiterer Verschattungs-Datensätze 36d-g erstellt (Block 44). Dafür werden im vorliegenden Beispiel die Pixel-Werte des Bereichs 34 genullt oder mit dem Wert Null ersetzt, die gemäß durch den Nutzer vorgegebenen Geometrie- und Relativorts-Daten 32 einer Fläche des zweifachen Einzelschatters 33-III entsprechen. Anders ausgedrückt wird für jeden durch den Nutzer vorgegebenen Drehwinkel βa-c des zweifachen Einzelschatters 33-III ein Verschattungs-Datensatz erstellt. Aus diesen vier erstellten Verschattungsdatensätzen 36d-g wird ein erster gemittelter Verschattungs-Datensatz berechnet 44, der als Basis für die Berechnung der vierten Helligkeit dient. Für die Berechnung der vierten Helligkeit wird über alle Pixel-Werte des ersten gemittelten Verschattungs-Datensatz integriert (Block 46).

Für die Berechnung einer fünften Helligkeit werden vier zusätzliche Verschattungs-Datensätze 36h-k erstellt (Block 44). Dafür werden in der vorliegenden Variante die Pixel-Werte des Bereich 34 genullt oder mit dem Wert Null ersetzt, die gemäß durch den Nutzer vorgegebenen Geometrie- und Relativorts-Daten 32 einer Fläche der ringförmigen Blende 33-II und des zweifachen Einzelschatters 33-III entspricht. Anders ausgedrückt wird für die entsprechende Schatter-Konstellation für jeden durch den Nutzer vorgegebenen Drehwinkeln βa-c des zweifachen Einzelschatters 33-III ein Verschattungs-Datensatz erstellt. Aus diesen vier erstellen Verschattungsdatensätzen 36h-k wird ein zweiter gemittelter Verschattungs-Datensatz berechnet (Block 44), der als Basis für die Berechnung der fünften Helligkeit dient. Für die Berechnung der fünften Helligkeit wird über alle Pixel-Werte des zweiten gemittelten Verschattungs-Datensatz integriert (Block 46).

Letztlich wird eine Vielzahl von Maßen einer Helligkeitsänderung berechnet (Block 48). In der dargestellten Variante jeweils ein Maß einer ersten, zweiten, dritten, vierten und fünften Helligkeitsänderung.

Jedes Maß der ersten bis fünften Helligkeitsänderung kann als Quotient aus Referenz-Helligkeit und einer entsprechenden berechneten Helligkeit gebildet werden - beispielsweise die Referenz-Helligkeit zu der erster Helligkeit oder die Referenz-Helligkeit zu der vierten Helligkeit.

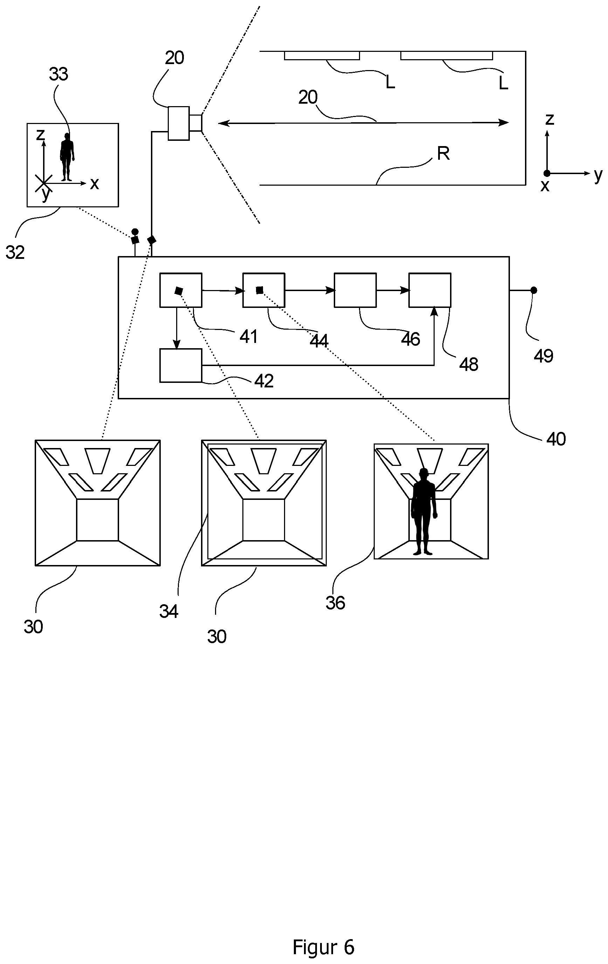

Fig.6 zeigt eine schematische Darstellung der Vorrichtung zur Anwendung in einem Raum. Die Vorrichtung weist eine Kamera 20 auf, die in der dargestellten Variante als Digital-Kamera 20 ausgebildet ist und ein Field-Of-View FOV aufweist. In dem Field-Of-View FOV ist in einem ersten Abstand 10 zur Digital-Kamera 20, entlang einer Längsachse y der Vorrichtung, eine Vielzahl von Lichtquellen L angeordnet, die ein Licht in Richtung der Digital-Kamera 20 und in einen Raum R abgeben. Die Digital-Kamera 20 ist dazu eingerichtet, die Lichtquelle L zu erfassen, Bilddaten 30 zu generieren und die Bilddaten 30 an eine Auswerteeinheit 40 zu senden. Die mit dem Bezugszeichen 30 bezeichneten Bilddaten zeigen die von der Digital-Kamera 20 erfasste Vielzahl von Lichtquelle L, d. h. insgesamt fünf Lichtquellen L. Zu erkennen ist, dass die Bilddaten 30 mit dem Field-of-View FOV der Digital-Kamera 20 korrespondieren. Die Vielzahl der Lichtquellen L in/an eine Decke des Raumes montiert.

Zusätzlich zu den Bilddaten 30 ist die Auswerteeinheit 40 dazu eingerichtet, Geometrie- und Relativorts-Daten 32 eines Abschatters 33 und der Vielzahl von Lichtquellen L zu empfangen. In der dargestellten Variante weisen die Geometrie- und Relativorts-Daten 32 eine Information über die Kontur des Abschatters 33, sowie über dessen Relativposition und dessen Relativlage in Bezug zu einer Längs-, Quer- und Hochachse y, x, z der Vorrichtung auf.

Die Information über Relativposition und Relativlage der Lichtquelle L in Bezug zu der Längs-, Quer-, und Hochachse y, x, z ist nicht grafisch in den Geometrie- und Relativorts-Daten 32 dargestellt.

Mit anderen Worten gibt der Nutzer vor, für welche Geometrie- und Relativorts-Daten 32 der Abschatter 33 in den Bereich der Bilddaten 30 projiziert werden soll. In der dargestellten Variante weist der Abschatter 33 die Kontur einer Person auf.

Hat der Nutzer diese Informationen vorgegeben erfolgt die weitere Verarbeitung der Bilddaten 30 und der Geometrie- und Relativorts-Daten 32 in der Auswerteeinheit 40.

Die dargestellte Auswerteeinheit 40 weist fünf Blöcke 41, 42, 44, 46, 48 auf, die die Verarbeitung der Geometrie- und Relativorts-Daten 32 und der Bilddaten 30 in der Auswerteeinheit 40 verdeutlichen sollen.

Zunächst wird ein Bereich 34 der Bilddaten 30 ausgewählt (Block 41). Zusätzlich kann vor oder nach der Bereichsauswahl eine Umrechnung des Bereich 34 oder der Bilddaten 30 erfolgen, um die später zu berechnende Referenz-Helligkeit oder erste Helligkeit vereinfacht zu berechnen, bspw. von RGB zu Lab. Dieser Bereich 34 kann entweder durch den Nutzer selbst in den Bilddaten 30 ausgewählt werden oder die Auswerteeinheit 40 kann auf Basis der Geometrie- und Relativorts-Daten 32 den Bereich 34 erkennen und jenen entsprechend auswählen, bspw. mit Methode des Image Processings.

Dieser Bereich 34 dient als Grundlage für die zu berechnende Referenz-Helligkeit und die erste Helligkeit.

Die Referenz-Helligkeit wird anhand des Bereichs 34 ermittelt (Block 42), indem über alle Pixel-Werte des Bereichs 34 integriert wird. Die in Block 42 berechnete Referenz-Helligkeit dient als Grundlage für eine Berechnung der Helligkeitsänderung.

Für die Berechnung der ersten Helligkeit wird zuerst ein erster Verschattungs-Datensatz 36 erstellt (Block 44). Dafür werden im vorliegenden Beispiel die Pixel-Werte des Bereich 34 genullt oder mit dem Wert Null ersetzt, die gemäß durch den Nutzer vorgegebenen Geometrie- und Relativorts-Daten 32 einer Fläche des personenförmigen Abschatters 33 entsprechen. Auf Basis dieses Verschattung-Datensatzes 36 wird eine erste Helligkeit berechnet (Block 46), indem über alle Pixel-Werte des Verschattung-Datensatzes 36 integriert wird.

Letztlich wird ein Maß einer erste Helligkeitsänderung berechnet (Block 48) und zwar auf Basis der berechneten ersten Helligkeit und der Referenz-Helligkeit und jenes Maß wird ausgegeben (Block 49).

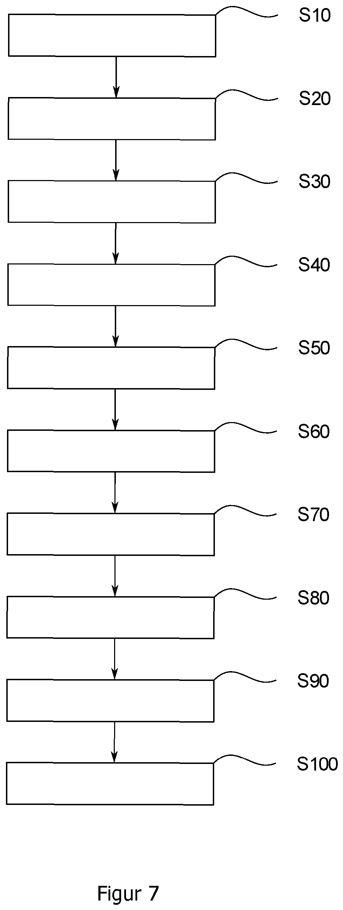

Fig. 7 zeigt ein Verfahren zur Vermessung zumindest einer entlang einer physisch abschatter-freien Messstrecke angeordneten Lichtquelle. Das dargestellte Verfahren umfasst die Schritte:
- Bereitstellen (S10) einer (Digital-) Kamera, die entlang der Messstrecke angeordnet ist, dazu eingerichtet, die zumindest eine Lichtquelle zu erfassen;
- Generieren (S20) von Bilddaten;
- Bereitstellen (S30) einer Auswerteeinheit;
- Senden (S40) der Bilddaten, insbesondere an die Auswerteeinheit;
- Empfangen (S50), mittels der Auswerteeinheit, der Bilddaten und durch einen Nutzer vorzugebende oder vorgegebene Geometrie- und Relativorts-Daten zumindest eines ersten Abschatters, insbesondere und der zumindest einen Lichtquelle;
- Berechnen (S60), mittels der Auswerteeinheit, einer Referenz-Helligkeit und/oder Referenz-Farbtemperatur basierend auf zumindest einem Bereich der Bilddaten, insbesondere und der Geometrie- und Relativorts-Daten;
- Erstellen (S70), mittels der Auswerteeinheit, zumindest eines ersten Verschattungs-Datensatzes basierend auf dem zumindest einen Bereich der Bilddaten und den vorgegebenen Geometrie- und Relativorts-Daten;
- Berechnen (S80), mittels der Auswerteeinheit, einer ersten Helligkeit und/oder einer ersten Farbtemperatur basierend auf dem zumindest einen ersten Verschattungs-Datensatz;
- Berechnen (S90), mittels der Auswerteeinheit, eines Maßes für eine erste Helligkeitsänderung basierend auf der Referenz-Helligkeit und der ersten Helligkeit, und/oder eines Maßes für eine erste Farbtemperaturänderung basierend auf der Referenz-Farbtemperatur und der ersten Farbtemperatur;
- Ausgeben (S100), mittels der Auswerteeinheit, des Maßes für eine erste Helligkeitsänderung und/oder des Maßes für die erste Farbtemperaturänderung.

## Patentansprüche

1. Vorrichtung zur Bestimmung einer Beleuchtungsänderung einer durch zumindest eine Lichtquelle (L) beleuchteten Messstrecke,
aufweisend:
- eine (Digital-) Kamera (20), dazu eingerichtet, die zumindest eine Lichtquelle (L) zu erfassen, Bilddaten (30) zu generieren und die Bilddaten, insbesondere an eine Auswerteeinheit (40), zu senden,
- eine Auswerteeinheit (40), dazu eingerichtet
- die Bilddaten (30) und durch einen Nutzer vorzugebende oder vorgegebene Geometrie- und Relativorts-Daten (32) zumindest eines ersten Abschatters (33a), insbesondere und der zumindest einen Lichtquelle (L), zu empfangen,
- eine Referenz-Helligkeit und/oder Referenz-Farbtemperatur basierend auf zumindest einem Bereich (34) der Bilddaten, insbesondere und der Geometrie- und Relativorts-Daten (32), zu berechnen (42),
- zumindest einen ersten Verschattungs-Datensatz (36a) basierend auf dem zumindest einen Bereich (34) der Bilddaten (30) und den vorgegebenen Geometrie- und Relativorts-Daten (32) zu erstellen (44),
- eine erste Helligkeit und/oder eine erste Farbtemperatur basierend auf dem zumindest einen ersten Verschattungs-Datensatz (36) zu berechnen (46),
- ein Maß für eine erste Helligkeitsänderung basierend auf der Referenz-Helligkeit und der ersten Helligkeit zu berechnen und/oder ein Maß für eine erste Farbtemperaturänderung basierend auf der Referenz-Farbtemperatur und der ersten Farbtemperatur zu berechnen (48) und das Maß für die erste Helligkeitsänderung und/oder das Maß für die erste Farbtemperaturänderung auszugeben (49).

2. Die Vorrichtung nach Anspruch 1, wobei die Auswerteeinheit (40) dazu eingerichtet ist
- durch einen Nutzer vorzugebende oder vorgegebene Geometrie- und Relativorts-Daten (32) zumindest eines zweiten Abschatters (33b) zu empfangen,
- zumindest einen zweiten Verschattungs-Datensatz (36b) basierend auf dem zumindest einen Bereich (34) der Bilddaten (30) und den vorgegebenen Geometrie- und Relativorts-Daten (32) zu erstellen,
- eine zweite Helligkeit und/oder eine zweite Farbtemperatur basierend auf dem zumindest einen zweiten Verschattungs-Datensatz (36b) zu berechnen (46),
- ein Maß für eine zweite Helligkeitsänderung basierend auf der Referenz-Helligkeit und der zweiten Helligkeit zu berechnen (48) und/oder ein Maß für eine zweite Farbtemperaturänderung basierend auf der Referenz-Farbtemperatur und der zweiten Farbtemperatur zu berechnen (48) und das Maß für die zweite Helligkeitsänderung und/oder das Maß für die zweite Farbtemperaturänderung auszugeben (49).

3. Die Vorrichtung nach Anspruch 1 oder 2, wobei die Auswerteinheit (40) dazu eingerichtet ist
- zumindest einen weiteren Verschattungs-Datensatz (36c) basierend auf dem zumindest einen Bereich (34) der Bilddaten (30) und den vorgegebenen Geometrie- und Relativorts-Daten (32) zu erstellen,
- eine weitere Helligkeit und/oder eine weitere Farbtemperatur basierend auf dem zumindest einen weiteren Verschattungs-Datensatz (36c) zu berechnen (46),
- ein Maß für eine weitere Helligkeitsänderung basierend auf der Referenz-Helligkeit und der weiteren Helligkeit zu berechnen (48) und/oder ein Maß für eine weitere Farbtemperaturänderung basierend auf der Referenz-Farbtemperatur und der weiteren Farbtemperatur zu berechnen und das Maß für die weitere Helligkeitsänderung und/oder das Maß für die weitere Farbtemperaturänderung auszugeben (49).

4. Die Vorrichtung nach einem der vorherigen Ansprüche, wobei
- die Geometriedaten der Lichtquelle (L) und/oder des ersten (33a) und/oder des zweiten (33b) Abschatters Information über eine Längs-, Quer- und/oder Hochachsausdehnung, insbesondere entlang einer Längs-, Quer und/oder Hochachse (y)(x)(z) der Vorrichtung, und/oder über einer Kontur aufweisen, und/oder
- der zumindest eine und/oder zweite Abschatter (33a)(33b) ausgebildet ist als eine Vielzahl von Abschattern, als Doppel-Abschatter, als, insbesondere kreisförmiger, Einzelschatter, als zweifacher Einzelschatter, als Blende, als Objekt mit einer, insbesondere kopfförmigen, handförmigen, körperförmigen, oder oberkörperförmigen, Kontur oder als geometrisches Objekt mit einer Kontur, die einer Kontur einer ebenen geometrischen Figur entspricht, oder als Operationswerkzeug.

5. Die Vorrichtung nach einem der vorherigen Ansprüche, wobei
- die Relativorts-Daten (32) Informationen aufweisen über
- eine Anzahl von Abschattern, insbesondere eine Anzahl der Vielzahl von Abschattern, und/oder
- eine Anzahl von, insbesondere möglichen oder durch einen Nutzer vorzugebende oder vorgegebene, Abschatter-Konstellationen aus zumindest zwei, drei oder vier Abschattern oder aus der Vielzahl von Abschattern.

6. Die Vorrichtung nach einem der vorherigen Ansprüche, wobei
- die Auswerteeinheit (40) dazu eingerichtet ist, durch einen Nutzer vorzugebende oder vorgegebene Relativorts-Daten (32) des zumindest einen ersten und/oder des zumindest einen zweiten Abschatters (33a)(33b) und/oder der Vielzahl von Abschattern (33-I)(33-II)(33-III) und der zumindest einen Lichtquelle (L) zu empfangen, und/oder
- die Relativorts-Daten (32) Informationen aufweisen über eine, insbesondere einzige, Relativlage oder eine Vielzahl von Relativlagen
- des zumindest einen ersten und/oder des zumindest einen zweiten Abschatters (33a)(33b) und/oder, insbesondere jeweils, der Vielzahl von Abschattern (33-I)(33-II)(33-III) gegenüber einer Längs-, Quer- und/oder Hochachse (y)(x)(z) und/oder gegenüber eines Abschatter- oder Abschatterkonstellation-Punkts, insbesondere eines Mittelpunkts oder Drehpunkts.

7. Die Vorrichtung nach einem der vorherigen Ansprüche, wobei
- die Relativorts-Daten (32) der Lichtquelle und des Abschatters Informationen aufweisen über eine, insbesondere einzige, Relativposition oder eine Vielzahl von Relativpositionen
- der (Digital-) Kamera (20) gegenüber der Lichtquelle (L),
- des zumindest einen ersten und/oder des zumindest einen zweiten Abschatters (33a)(33b) gegenüber der Lichtquelle (L) und/oder gegenüber der (Digital-) Kamera (20),
- des zumindest einen ersten und/oder des zumindest einen zweiten Abschatters (33a)(33b) zueinander,
- der Vielzahl von Abschattern (33-I)(33-II)(33-III) jeweils zueinander, und/oder
- die Relativorts-Daten (32) des Abschatters Informationen aufweisen über Lichteigenschaften, insbesondere (graduelle) Opazität, oder Reflexions- und/oder Transmissionseigenschaften, für zumindest einen Teil oder die (ganze) Oberfläche, des zumindest einen ersten und/oder des zumindest einen zweiten Abschatters und/oder über eine auf einen Rand des zumindest einen ersten und/oder des zumindest einen zweiten Abschatters, insbesondere während der Erstellung des Verschattungs-Datensatzes, anzuwendende Gewichtungsfunktion.

8. Die Vorrichtung nach einem der vorhergehenden Ansprüche, wobei die Auswerteinheit (40) dazu eingerichtet ist
- eine durch den Nutzer vorzugebende Vielzahl von Geometrie- und Relativorts-Daten der zumindest einen ersten und/oder des zumindest einen zweiten Abschatters zu empfangen,
- eine Vielzahl von Verschattungs-Datensätzen zu erstellen, wobei jeder der Vielzahl von Verschattungs-Datensätzen basierend auf dem zumindest einen Bereich der Bilddaten und, insbesondere jeweils, einer der Vielzahl von Geometrie- und Relativorts-Daten des zumindest einen ersten und/oder des zumindest einen zweiten Abschatters erstellt wird,
- eine Vielzahl von Helligkeiten und/oder eine Vielzahl von Farbtemperaturen zu berechnen, wobei jede der Vielzahl von Helligkeiten und/oder jede der Vielzahl von Farbtemperaturen basierend auf, insbesondere jeweils, einem der Vielzahl von Verschattungs-Datensätzen und, insbesondere jeweils, einem der Vielzahl von Geometrie- und Relativorts-Daten berechnet wird, und
- eine Vielzahl von Maßen für Helligkeitsänderungen und/oder eine Vielzahl von Maßen für Farbtemperaturänderungen zu berechnen und auszugeben, wobei jedes Maß der Vielzahl von Maßen für Helligkeitsänderungen basierend auf der Referenz-Helligkeit und, insbesondere jeweils, einer der Vielzahl von Helligkeiten oder einer von dem Nutzer vorzugebenden oder vorgegebenen Auswahl der Vielzahl von Helligkeiten berechnet wird und/oder wobei jedes Maß der Vielzahl von Maßen für Farbtemperaturänderungen basierend auf der Referenz-Farbtemperatur und, insbesondere jeweils, einer der Vielzahl von Farbtemperaturen oder einer von dem Nutzer vorzugebenden oder vorgegebenen Auswahl der Vielzahl von Farbtemperaturen berechnet wird.

9. Die Vorrichtung nach einem der vorhergehenden Ansprüche, wobei
- die Auswerteinheit (40) dazu eingerichtet ist, in dem Verschattungs-Datensatz (36) zumindest einen Abschnitt zu bestimmen und dessen Helligkeitswerte und/oder Farbwerte zu korrigieren, insbesondere zu reduzieren, basierend auf den Bilddaten (30) und den einzugebenden Geometrie- und Relativorts-Daten (32) und/oder-
- die erste, die zweite, die weitere oder die Vielzahl von Helligkeitsänderungen als Helligkeitsminderung ausgebildet ist und/oder
- die erste, die zweite, die weitere oder die Vielzahl Farbtemperaturänderungen als Farbtemperaturanpassung ausgebildet ist.

10. Die Vorrichtung nach einem der vorherigen Ansprüche, wobei die Lichtquelle (L) als Kunstlichtquelle oder als Leuchte, insbesondere als Operationsleuchte, ausgebildet ist.

11. Die Vorrichtung nach einem der vorherigen Ansprüche, wobei der Bereich (34) der Billdaten mit einem Field-of-View (FOV) der (Digital-)Kamera (20), insbesondere mit einem durch den Nutzer zu wählenden oder gewählten Ausschnitt des Field-of-Views, korrespondiert.

12. Die Vorrichtung nach einem der vorherigen Ansprüche, wobei der Bereich (34) der Bilddaten (30) mit einer, insbesondere gesamten und/oder durch den Nutzer zu wählenden oder gewählten, Lichtaustrittsfläche der zumindest einen Lichtquelle (L) korrespondiert.

13. Die Vorrichtung nach einem der vorherigen Ansprüche, wobei die Auswerteeinheit (40) dazu eingerichtet ist, zumindest eine durch den Nutzer vorzugebende oder vorgegebene, insbesondere Mittelwert-, Berechnungsvorschrift für zumindest zwei, drei, vier, fünf oder eine Vielzahl von Verschattungs-Datensätzen (36) und die erste, zweite, dritte, weitere und/oder Vielzahl von Helligkeiten auf Basis des Ergebnisses der Berechnungsvorschrift zu berechnen (46); und/oder zumindest zwei, drei, vier, fünf oder eine Vielzahl von Verschattungs-Datensätzen (36) und die erste, zweite, dritte, weitere und/oder die Vielzahl von Farbtemperaturen auf Basis des Ergebnisses der Berechnungsvorschrift zu berechnen.

14. Die Vorrichtung nach einem der vorhergehenden Ansprüche, wobei die (Digital-) Kamera als Farb-, insbesondere RGB-, Kamera, oder hyperspektrale, insbesondere 2D-Sensor, Kamera ausgebildet ist und/oder
- die eine Auswerteeinheit, dazu eingerichtet
- die Bilddaten und durch einen Nutzer vorzugebende oder vorgegebene Geometrie- und Relativorts-Daten zumindest eines ersten Abschatters, insbesondere und der zumindest einen Lichtquelle, zu empfangen,
- eine Referenz-Helligkeitsverteilung und/oder eine Referenz-Farbtemperaturverteilung basierend auf zumindest einem Bereich der Bilddaten, insbesondere und der Geometrie- und Relativorts-Daten, zu berechnen,
- zumindest einen ersten Verschattungs-Datensatz basierend auf dem zumindest einen Bereich der Bilddaten und den vorgegebenen Geometrie- und Relativorts-Daten zu erstellen,
- eine erste Helligkeitsverteilung und/oder eine erste Farbtemperaturverteilung basierend auf dem zumindest einen ersten Verschattungs-Datensatz zu berechnen,
- ein zumindest, insbesondere auf zumindest ein Pixel der Kamera bezogenes, ortsaufgelöstes Maß für eine erste Helligkeitsänderung basierend auf der Referenz-Helligkeitsverteilung und der ersten Helligkeitsverteilung zu berechnen und/oder ein zumindest, insbesondere auf das oder ein Pixel der Kamera bezogenes, ortsaufgelöstestes Maß für eine erste Farbtemperaturänderung basierend auf der Referenz-Farbtemperaturverteilung und der ersten Farbtemperaturverteilung zu berechnen und das Maß für die erste Helligkeitsänderung und/oder das Maß für die erste Farbtemperaturänderung auszugeben.

15. Verfahren zur Bestimmung einer Beleuchtungsänderung einer durch zumindest eine Lichtquelle beleuchteten Messstrecke, umfassend die Schritte:
- Bereitstellen (S10) einer (Digital-) Kamera, die entlang der Messstrecke angeordnet ist, dazu eingerichtet, die zumindest eine Lichtquelle zu erfassen;
- Generieren (S20) von Bilddaten;
- Bereitstellen (S30) einer Auswerteeinheit;
- Senden (S40) der Bilddaten, insbesondere an die/eine Auswerteeinheit;
- Empfangen (S50), mittels der Auswerteeinheit, der Bilddaten und durch einen Nutzer vorzugebende oder vorgegebene Geometrie- und Relativorts-Daten zumindest eines ersten Abschatters, insbesondere und der zumindest einen Lichtquelle;
- Berechnen (S60), mittels der Auswerteeinheit, einer Referenz-Helligkeit und/oder Referenz-Farbtemperatur basierend auf zumindest einem Bereich der Bilddaten, insbesondere und der Geometrie- und Relativorts-Daten;
- Erstellen (S70), mittels der Auswerteeinheit, zumindest eines ersten Verschattungs-Datensatzes basierend auf dem zumindest einen Bereich der Bilddaten und den vorgegebenen Geometrie- und Relativorts-Daten;
- Berechnen (S80), mittels der Auswerteeinheit, einer ersten Helligkeit und/oder einer ersten Farbtemperatur basierend auf dem zumindest einen ersten Verschattungs-Datensatz;
- Berechnen (S90), mittels der Auswerteeinheit, eines Maßes für eine erste Helligkeitsänderung basierend auf der Referenz-Helligkeit und der ersten Helligkeit und/oder eines Maßes für eine erste Farbtemperaturänderung basierend auf der Referenz-Farbtemperatur und der ersten Farbtemperatur;
- Ausgeben (S100), mittels der Auswerteeinheit, des Maßes für eine erste Helligkeitsänderung und/oder des Maßes für die erste Farbtemperaturänderung.
